Europäisches Patentamt

European Patent Office

Office européen des brevets

(11) Publication number: **0 414 569 A2**

# EUROPEAN PATENT APPLICATION

(21) Application number: **90309337.5**

(22) Date of filing: **24.08.90**

(51) Int. Cl.5: **C07D 301/03**, C07D 303/48

(30) Priority: **25.08.89 US 401069**
**28.06.90 US 545040**

(43) Date of publication of application:
**27.02.91 Bulletin 91/09**

(84) Designated Contracting States:
**AT BE CH DE DK ES FR GB GR IT LI LU NL SE**

(71) Applicant: **E.I. DU PONT DE NEMOURS AND COMPANY**
**1007 Market Street**
**Wilmington Delaware 19898(US)**

(72) Inventor: **Lawson, James Robert**
**10 Hickory Lane**
**Marietta, Ohio 45750(US)**

(74) Representative: **Myerscough, Philip Boyd et al**
**J.A.Kemp & Co. 14, South Square Gray's Inn**
**London, WC1R 5EU(GB)**

(54) Three-liquid-phase epoxidation of perfluoroolefins.

(57) A process for converting a perfluoroolefin to its epoxide by agitation at reaction conditions that maintain three liquid phases - an aqueous phase comprising hypohalite ions, an epoxide phase comprising the bulk of the epoxide and essentially no phase transfer catalyst, and a reaction phase comprising an organic liquid in which a phase transfer catalyst is soluble and which has a higher affinity for perfluoroolefin than for its epoxide (partition coefficient greater than 1.3).

EP 0 414 569 A2

# THREE-LIQUID-PHASE EPOXIDATION OF PERFLUOROOLEFINS

## FIELD OF INVENTION

The present invention relates to a process for converting perfluoroolefins to their epoxides. It is especially useful in the epoxidation of hexafluoropropylene (HFP) to produce hexafluoropropylene epoxide (HFPO).

## BACKGROUND

Hexafluoropropylene epoxide (HFPO) is a versatile intermediate which can be converted to inert oils and to vinyl monomers, particularly perfluoromonomers. It has been made from hexafluoropropylene (HFP) by several processes, most of which have selectivity [1] in the range of 60-80%. Millauer, Chem Ing Tech 52, 53-55 (1980) reports an electrochemical process which gives a selectivity of 90%, but the current yield is only about 65% and the conversion [2] of HFP is about 65-70%. Kolenko et al., Izv. Akad. Nauk, SSR, Ser. Khim. (1979) No. 11, pps. 2509-2512, made HFPO in 52% selectivity in a one-liquid-phase system containing acetonitrile and aqueous NaOCl and no phase transfer catalyst.

Of particular note is the process taught in EP 64293 and U.S. 4,902,810 (Ikeda et al.). It is a hypochlorite oxidation employing two liquid phases, using a phase transfer catalyst. The best selectivity among 55 examples was in Example 1, carried out in a 50 ml pressure bottle. That example stated a 96% conversion of HFP and an HFPO selectivity of 84% were obtained.

The main problem Ikeda et al. were trying to solve was to find a process for converting HFP to HFPO in very high conversion, with as high a selectivity as possible. This was presumably because they wanted to avoid having to separate HFP from HFPO. However, HFP and HFPO can be separated by extractive distillation and, in some reactions, HFPO can be used without separating it from HFP, which is inert in many of the reactions of HFPO.

The problem of the present invention is to obtain the maximum selectivity at moderate conversion of HFP, using a minimum amount of phase transfer catalyst.

These differences in problems led Ikeda et al. to collect HFPO in the organic phase of a two-liquid-phase system, while the present invention collects HFPO in a 3rd liquid phase. Further, the present invention teaches how to select an organic liquid for use as one of the three liquid phases in such a way that HFPO is less soluble in that liquid than is HFP, thus protecting HFPO from attack by hypochlorite even more effectively than did Ideda et al.

Ikeda et al. named 25 specific organic liquids. Their examples used F-113 ($CF_2ClCFCl_2$), perfluorodimethyl cyclobutane, $CCl_4$, $CHCl_3$, $CH_2Cl_2$, $ClCH_2CH_2Cl$, n-hexane, diisopropyl ether, and a 1benzene/9hexane mixture. The organic liquids for use in the present invention are selected by the partition coefficient described below, which is designed to provide more HFP0 than HFP0 in the organic liquid phase at reaction conditions. In the prosecution of U.S. 4,902,810, Ikeda et al. dropped coverage of processes using low pH, requiring that the reaction be carried out "in the presence of an inorganic base." In the present invention, high pH can be used, but pH in the range 7.8-9.0, a range dropped by Ikeda et al. in U.S. 4,902,810, is especially preferred.

Example 1 of Ikeda et al. and most of its other examples used $CF_2ClCFCl_2$ as the organic solvent. The use of such an organic solvent is undesirable because this chlorofluorocarbon is the type of compound believed to cause problems in the ozone layer of the atmosphere.

Example 1 of Ideda et al. and nearly all its other examples use at least twice the stoichiometric amount of hypochlorite, with the result that much hypochlorite is wasted and a highly alkaline oxidizing waste stream must be disposed of, creating a safety problem.

In the present invention, the use of chlorofluorocarbons is easily avoided. Also, the amount of waste hypochlorite and caustic is sharply reduced.

[1]Selectivity is defined as moles of product produced divided by moles of starting material consumed, expressed as a percentage.

[2]Conversion is defined as moles of starting material converted divided by moles of starting material charged, expressed as a percentage.

The selectivity to HFPO is, in the better examples of the present invention, equal to the best example of Ikeda et al.

## SUMMARY OF THE INVENTION

The present invention is a process for converting a perfluoroolefin to its epoxide by agitation of a system of three liquid phases - an aqueous phase, an epoxide phase and a reaction phase -in the presence of a phase transfer catalyst for a sufficient time to convert a substantial part of the perfluoroolefin to its epoxide. The preferred perfluoroolefin is hexafluoropropylene.

The aqueous phase is a solution of a water-soluble metal hypohalite, containing 0.001 to 25% available halogen by weight, at a pH that assures high selectivity at the highest attainable conversion. The preferred hypohalite is sodium hypochlorite.

The epoxide phase contains the bulk of the epoxide, some perfluoroolefin, and as small an amount of catalyst and the organic liquid of the reaction phase as possible. It may contain an organic solvent for the epoxide, the solvent being substantially immiscible with both the aqueous and reaction phases

The reaction phase comprises an organic liquid, in which the catalyst is soluble and in which the olefin is more readily soluble than its epoxide at reaction conditions. It contains sufficient olefin to provide a reasonable reaction rate. The selection of an appropriate organic liquid is carried out by measuring a partition coefficient as described below. Preferably, the organic liquid is toluene.

The phase transfer catalyst can be any known phase transfer catalyst that is resistant to oxidation and halogenation at reaction conditions. It should be substantially more soluble in the reaction phase than in the epoxide phase. Preferably, the catalyst is essentially insoluble in the epoxide phase. The literature, including Ikeda et al., teaches several classes of catalysts which are stated to be functional and to be substantially equivalent to each other.

Reaction conditions should be such that three phases are maintained as the reaction progresses. It is particularly important that the reaction phase organic liquid not be oxidized or halogenated to a compound in which the epoxide would be as soluble as the starting olefin.

The process may be batch or continuous. The reaction temperature should be high enough that no component is substantially a solid and low enough that no component is substantially a gas. The reaction pressure should be such that three liquid phases are present. The pH should be such that epoxide hydrolysis is minimized. Conversion and selectivity can be increased and phase transfer catalyst concentration can be decreased by operating at about pH 8.2 instead of 12. Control of pH in this range can be simplified by using a buffer such as an alkali metal carbonate.

The perfluoroolefin epoxide is recovered from the reaction mixture that results.

## DETAILS OF THE INVENTION

It is particularly important that the reaction system of the present invention contain three liquid phases. While not wishing to be inappropriately limited, it is believed that the phase transfer catalyst helps transport the oxidant from the aqueous phase into the reaction phase where it reacts with the olefin. The epoxide product, being less soluble than the reactant perfluoroolefin in the reaction phase, separates into a third epoxide phase where it is believed to be protected from overoxidation by the oxidant due to the substantial insolubility of the catalyst in that phase.

A wide range of perfluoroolefins, preferably containing at least three carbon atoms, can be converted into their corresponding epoxides by the process of this invention. Examples demonstrate applicability to perfluorocyclohexene, perfluorocyclopentene, perfluoroheptene-1, and hexafluoropropylene. The last of these is of the greatest industrial interest. (Other fluorinated olefins can be converted according to the process so long as the carbons composing the double bond are only bonded to another carbon or a fluorine.) Cyclic perfluoroolefins and terminally unsaturated perfluoroolefins are preferred. Terminally unsaturated perfluoroolefins are most preferred, especially hexafluoropropylene.

The oxidant can be any hypohalite, with hypochlorite being the most readily available. For the purposes of this application, hypochlorite will be referenced since it is the most preferred but other hypohalites should be implied as being included.

The hypochlorite concentration in the reaction may be 0.001 to 25 wt % as chlorine in the aqueous phase depending upon which organic liquid is used and the conditions of reaction. It is important not to substantially alter the ability of the organic liquid to form two phases with the perfluoroolefin and its epoxide.

To minimize undesired byproducts from hypochlorite attack on the organic liquid or catalyst and to reduce the amount of hypochlorite present in the aqueous waste, it is desirable to maintain the hypochlorite concentration at a low level, preferably 1.5 wt % or less as chlorine in the aqueous phase. For rate of reaction, a concentration of 0.01 wt. % or higher as chlorine is preferred.

The ratio of hypohalite to perfluoroolefin, particularly in continuous reactions, should be limited to a molar ratio of less than 1.2 to 1 in order to minimize attack on organic liquid and catalyst and to minimize hypohalite content of aqueous waste streams. The molar ratio preferably should be 0.5 to 1 or higher to provide acceptable conversion.

The phase transfer catalyst can be any known phase transfer catalyst that is resistant to oxidation and halogenation under reaction conditions. It should be much more soluble in the reaction phase than in the epoxide phase. Preferably, the catalyst should be essentially insoluble in the epoxide phase. The literature, including Ikeda et al., teaches several classes of catalysts which are stated to be functional and to be substantially equivalent to each other. Ikeda et al., incorporated by reference, discloses a lengthy list of such phase transfer catalysts including those selected from the class consisting of

a) quaternary ammonium salts,
b) quaternary phosphonium salts,
c) quaternary arsonium salts,
d) sulphonium salts,
e) onium salts,
f) crown ethers, and
g) lipophilic complexing agents. The preferred catalysts are organic-soluble quaternary ammonium salts, more preferably coco benzyl bis(betahydroxypropyl) ammonium chloride [3].

The concentration of the phase transfer catalyst or catalysts is not critical. The optimum catalyst concentration depends on the structure of the catalyst and the selection of the organic liquid. Increased catalyst concentration increases reaction rate and conversion. At excessively high catalyst concentrations, however, selectivity decreases. With the coco benzyl bis(betahydroxypropyl) ammonium chloride and toluene used in the examples, a suitable concentration is between 0.001 and 0.2 grams per gram (g/g) HFP, preferably between 0.005 and 0.05 g/g HFP, and most preferably between 0.008 and 0.02 g/g HFP. For each catalyst and organic liquid, routine experimentation will determine the best balance between selectivity and conversion for the perfluoroolefin being epoxidized. Sufficient catalyst to achieve the optimum balance should be used.

Certain organic liquids show more affinity for perfluoroolefins than for their epoxides and they are desired for use in this invention. For example, toluene becomes immiscible with HFP at concentrations of 20-40 weight percent (wt %) and with HFPO at concentrations of 3-6 wt %. Miscibility is sharply affected by temperature, and toluene, for example, becomes completely miscible with HFP at temperatures greater than $6°$ C. With mixtures of HFP and HFPO as would exist in a continuous back-mixed reactor, immiscibility exists at higher temperatures. It is believed that organic liquids which are substantially halogenated will not form two phases with HFP or HFPO. This has been demonstrated in the case of $CF_2ClCFCl_2$. Therefore, a hydrogen-containing organic liquid is used.

The reaction phase organic liquid or solvent should be resistant to reaction with any component of the system at reaction conditions, particularly if such reaction would alter the preference for starting olefin (HFP, for example) over the preference for its epoxide (HFPO, for example).

A simple method of determining this preference (using HFP over HFPO as an example of other olefins and their epoxides covered by this invention) is to determine the partition coefficient for the system HFPO-HFP-organic liquid. Determination of the partition coefficient (alpha) is carried out by passing a mixture of HFP and HFPO gas through the organic liquid at atmospheric pressure while agitating vigorously and maintaining a constant temperature. The gas is passed through the liquid long enough to ensure that the liquid is completely saturated with the gases and to let the gas phase composition equilibrate to the feed composition. The organic liquid phase is then sampled and gas chromatography is used to determine the amount of the gaseous components which had been dissolved in the liquid phase. The partition coefficient, alpha, is then calculated by the following equation:

$$\text{alpha} = \frac{([HFPO]/[HFP]) \text{ gas phase}}{([HFPO]/[HFP]) \text{ liquid phase}}$$

---

[3] The term "coco" refers to a mixture of alkyl groups containing 12-18 carbon atoms.

An HFP-HFPO partition coefficient greater than 1.0 suggests that the organic liquid dissolves HFP more readily than it dissolves HFPO. Preferably, the partition coefficient for the present invention should be greater than 1.3, more preferably 1.9 or greater, and most preferably 2.3 or greater at the temperature of operation. Higher partition coefficients suggest that the organic liquid will perform well in the oxidation of HFP to HFPO provided it fulfills the other requirements described herein.

Using this method, partition coefficients were determined for a number of organic liquids at -15° C and 0° C, with the results shown below:

| Solvent | Alpha at -15° C | Alpha at 0° C |
|---------|--------------|-------------|
| FC-75[4] | 1.0 | 1.0 |
| n-decane | 1.6 | 1.3 |
| chlorobenzene | 1.9 | 2.1 |
| dichlorobenzene | 2.0 | 2.1 |
| mesitylene | 2.3 | 2.2 |
| anisole | 2.4 | 2.5 |
| toluene | 2.6 | 2.3 |

[4] Perfluoro(butyl tetrahydrofurane)

For an HFP-to-HFPO reaction, toluene is the preferred solvent.

Further discussion of the organic liquids employed in the reaction phase of this invention is found in the Examples where oxidation experiments using the above liquids are set forth.

The organic phases must be substantially immiscible or sparingly miscible with the aqueous phase.

The epoxide phase is rich in epoxide. That is, it contains the bulk of the epoxide formed in the reaction. It also contains some perfluoroolefin, particularly at the beginning of the reaction, and as small an amount of catalyst and the organic liquid of the reaction phase as possible. Optionally, it may contain known epoxide solvents, the solvent being substantially immiscible in both the aqueous and reaction phases. Suitable epoxide solvents are typically perhalogenated or perfluorinated compound other than the perfluoroolefin or its epoxide. The purpose of the epoxide solvent is to increase selectivity by facilitating reduction of epoxide concentration in the reaction phase and increasing it in the epoxide phase. As such, the epoxide solvent is most useful when the partition coefficient of the reaction-phase organic liquid is relatively low, say 1.3 to 1.8. The epoxide solvent would preferably be omitted at partition coefficients of 1.9 or higher.

The process of the invention can be run in a variety of reactor configurations including but not limited to batch, semi-batch, continuous back-mixed tank, and continuous plug flow. The type of reactor is not critical.

Reaction temperature is critical only in the sense that three liquid phases must be present during the reaction, but it is significant in three ways. First, when using an aqueous medium, the minimum temperature which can be reached without freezing the water is about -25° C, even with maximum addition of NaCl to suppress the freezing point. (Other salts such as CaCl$_2$ suppress the freezing point even more.) Second, increasing the temperature appears to decrease the selectivity. Finally, as stated above, the temperature must be maintained in a region where three liquid phases exist. The last two considerations limit maximum temperature to about 60° C. The temperature range for HFP oxidation is preferably from -20° to +15° C, more preferably -15° to +5° C. Operation at the higher end of the preferred range is particularly suited to continuous operation.

Reaction pressure is not critical except that three liquid phases must be present. Therefore pressure should be at least as high as the sum of the partial pressures of the system at the particular temperature of operation. The pressure is preferably between 1 and 20 atmospheres, with 2-6 atmospheres being preferred for the toluene/HFP system.

The pH of the system must be greater than 7, but high pH can cause hydrolysis of HFPO and other perfluoroepoxides. Therefore the pH should be between 7 and 14, preferable 7.5-11. The more preferred pH is 7.5-9.6, especially 7.8-9.0, most preferably 8.0-8.5. Control of pH is easier below pH 9.6, and the reduction of the pH of the aqueous waste stream minimizes the environmental impact of waste disposal. In the more preferred range, pH control is further simplified by using a buffer such as Na$_2$CO$_3$ or the equivalent.

At pH 7.5-9.6, equilibrium data show that both HOCl and OCl$^-$ are present. However, the more preferred pH range may involve a different mechanism from operation at pH 10-13 (see JACS 105 7672-

5

7676 [1983]). This reference suggests that ClO•, a free radical, is the reactive intermediate in the reaction under phase transfer catalysis conditions at pH 7.5-9 of hypochlorite with either alkenes, toluene, 11 alkanes, or anisole. These workers did not use fluoroolefins and their reaction temperature, reaction time, and phase transfer catalyst were different from those of the present work. The prior art work with toluene caused almost 10% conversion of toluene, while the present work found only 1-4% conversion of toluene at pH about 12. In any event, operation at the preferred pH range provides excellent conversion and selectivity even with a lower concentration of catalyst than is required at higher pH to give comparable conversion.

HFPO may be recovered from the reaction mixture in any of several ways, depending on the exact system. For example, the third liquid phase can be separated from the reaction mixture by decantation and the reactant and product recovered by distillation. In the case in which the third phase consists only of HFP and HFPO, the reactant and product can be recovered from the reaction mixture simply by reducing the pressure and allowing the third phase to evaporate to a gas. When using toluene or a similar high-boiling solvent as the reaction phase, as is done in this invention, this method of product recovery by pressure reduction is simple and highly effective, yielding a nearly pure HFPO/HFP gas.

## EXAMPLES

## EXAMPLE 1

A 1 liter agitated glass autoclave was charged with the following ingredients:
50 grams of toluene
14.95 grams of KOH
591 grams of aqueous NaOCl (4.12% available Cl$_2$ by titration)
0.5 grams[5] catalyst, coco benzyl bis(beta-hydroxypropyl) ammonium chloride.

The reactor was cooled to -6°C and 50.08 grams of HFP were added. Agitation was started and continued for 60 minutes. The average temperature during the run was -1.4°C. At the end of the reaction the stirrer was stopped. The volatile contents of the reactor were removed slowly and the condensable portion was condensed in a dry ice trap. HFP and HFPO left in the reactor after the pressure had been reduced to atmospheric were removed by sparging with helium. The condensable portion was analyzed and found to contain 52.8 mole percent (mole %) HFP and 47.2 mole % HFPO. The weight of the condensable portion was 37.15 grams. This represents a conversion of 62.7 % and a selectivity of 53.2%.

The gas and organic phase analyses were carried out by gas chromatography.

The organic phase left in the reactor was analyzed and found to be 99.7% toluene. A peak of 0.04 area % was found which represents toluene reaction products with the oxidant. The aqueous phase was titrated and found to contain 0.22% available Cl$_2$.

## EXAMPLE 2

The same procedure as in Example 1 was used except that no base was initially added. The pH of the reacting mixture was measured and controlled at about pH 9 by addition of 3N KOH. The reactor was cooled to 0.6°C and 50.97 grams of HFP were added. The average temperature during the run was 0.4°C. Recovery of products after sixty minutes of agitation yielded 38.17 grams, with a composition of 52.0 mole % HFP and 48.0 mole % HFPO. This represents a conversion of 63.0 % and a selectivity of 54.3 %. The toluene phase contained 0.1 area % byproducts. The hypochlorite concentration in the aqueous phase was not measured. This example shows a modest improvement in selectivity when the pH was controlled by addition of base.

## EXAMPLE 3

The same procedure as in Example 2 was used except that the sodium hypochlorite solution was

[5]In the examples unless otherwise indicated, the catalyst is charged as a 60% aqueous solution which also contains some of the starting materials for synthesis of the catalyst. The weight of catalyst is the weight of active ingredient.

presaturated with sodium chloride to prevent freezing, and the reactor was precooled to -7°C, after which 50.44 grams of HFP were added. During 60 minutes of agitation, the temperature averaged -13.5°C. Recovery of products yielded 45.17 grams with a composition of 45.1 mole % HFP and 54.9 mole % HFPO. This represents a conversion of 61.8 % and a selectivity of 75.1 %. The toluene phase contained 0.1 area % byproducts. The aqueous phase contained 0.06 % available $Cl_2$. This example shows the advantage of operating at lower temperatures where the miscibility between toluene and HFPO is much smaller.

## EXAMPLE 4

The procedure was the same as in Example 3 except that hypochlorite solution was added continuously during the run instead of being added at the start. pH values were not recorded. The reactor was precooled to -17°C, after which 50.92 grams of HFP were added. During 60 minutes of agitation, the temperature averaged -13.6°C. Recovery of products yielded 46.25 grams with a composition of 38.3 mole % HFP and 61.7 mole % HFPO. This represents a conversion of 67.1 % and a selectivity of 78 %. The toluene phase contained 0.1 area % byproducts. The aqueous phase contained 0.01 % available $Cl_2$. This example shows the benefit of adding hypochlorite continuously versus batchwise.

## EXAMPLE 5

The procedure of Example 4 was used except that the total agitation time was cut to 15 minutes and the hypochlorite addition rate was quadrupled to allow addition of the same total amount of hypochlorite. The amount of HFP added was 51.72 grams. During the 15 minute agitation period, reactor temperature averaged -8.6°C. Recovery of products yielded 45.55 grams with a composition of 48.4 mole % HFP and 51.6 mole % HFPO. This represents a conversion of 59.4 % and a selectivity of 72.2 %. The toluene phase contained 0.1 area % byproducts. The aqueous phase contained 0.01 % available $Cl_2$. This example shows that decreasing the reaction time by as much as four-fold has a modest effect on conversion and selectivity.

## EXAMPLES 6-9

The procedure of Example 4 was used except the amount of phase transfer catalyst was varied.

| Example | Catalyst, g | HFP, g | Conversion | Selectivity |
|---------|-------------|--------|------------|-------------|
| 6 | 0.06 | 51.99 | 15.4% | 48.2% |
| 7 | 0.3 | 53.08 | 47.9% | 77.6% |
| 8 | 0.6 | 52.26 | 50.9% | 73.0% |
| 9 | 3.0 | 51.71 | 78.2% | 60.5% |

The pH for Example 6 averaged 11 and the pH for Example 9 averaged 8. No data are available for Examples 7 and 8.

These examples show that there is an optimum amount of catalyst to achieve high selectivity, but that more catalyst leads to higher conversion.

## EXAMPLES 10-13

The procedure of Example 4 was used except the amount of toluene was varied.

| Example | Toluene, g | HFP, g | Conversion | Selectivity |
|---|---|---|---|---|
| Comp. 1 | 0 | 51.80 | 13.7% | 49.6% |
| 10 | 6 | 52.01 | 54.1% | 60.1% |
| 11 | 12.5 | 50.06 | 55.3% | 84.6% |
| 12 | 25 | 51.34 | 48.3% | 77.8% |
| 7 | 50 | 53.08 | 47.9% | 77.6% |
| 13 | 100 | 52.10 | 55.5% | 80.5% |

The average pH values for the six runs were 10, 12, 12, 11, not available, and 10, respectively.

These examples show that very poor results are obtained in the absence of an organic solvent and that results are not so good when, as in Example 10, the amount of toluene is too small to put the system into the three liquid phase region at the beginning of the reaction (the system becomes three-phase as the reaction progresses since HFPO is less soluble in toluene than HFP). An amount of toluene just great enough to put the system initially in the three-phase region (Ex. 11) greatly improves the results, which are then relatively insensitive to additional toluene.

## EXAMPLES 14-17

The procedure of Example 4 was used except that the nature of the solvent was varied.

| Example | Solvent | HFP, g | Conversion | Selectivity |
|---|---|---|---|---|
| Comp. 2 | FC-75 | 51.39 | 45.5% | 11.7% |
| Comp. 3 | n-decane | 51.73 | 17.3% | 43.6% |
| 14 | chlorobenzene | 52.57 | 58.8% | 70.2% |
| 15 | dichlorobenzene | 52.02 | 49.7% | 76.2% |
| 16 | mesitylene | 51.38 | 56.1% | 80.7% |
| 17 | anisole | 45.01 | 56.0% | 68.1% |
| 7 | toluene | 53.08 | 47.9% | 77.6% |

The average pH values for these runs were 11, 11, 10, 10, 11, 9 and not available, respectively.

In the case of FC-75, alpha is low so selectivity is poor. In the case of n-decane, the catalyst is not sufficiently soluble to allow the reaction to occur at an appreciable rate so the conversion is low. Selectivity, in the case of n-decane, is also marginal since the alpha of n-decane is low.

## EXAMPLES 18-20

Other perfluorinated olefins which were tested were liquids at atmospheric pressure and room temperature, while HFP is a gas. A 200 ml jacketed glass reactor was used at atmospheric pressure. Hypochlorite was added at equimolar amounts to the olefin. Toluene was added as the organic solvent and the catalyst used was coco benzyl bis(betahydroxypropyl) ammonium chloride. The pH of the batch reaction was controlled at 9 to 11 by periodic addition of NaOH. The reaction was terminated and the number of phases noted. Each organic phase was analyzed by infrared spectroscopy to determine the percentage recovery of fluorocarbon and the conversion of the olefin to epoxide.

Olefin A is perfluorocyclohexene, olefin B is perfluorocyclopentene and olefin C is perfluoroheptene-1. All three experiments showed three liquid phases. Conversion and selectivity are infrared area %.

| Ex | Feed | Solvent ml/g Alkene | Catalyst % of Alkene | Run Time (hrs) | Conversion | Selectivity |
|----|------|---------------------|----------------------|----------------|------------|-------------|
| 18 | A | 0.9 | 0.8 | 1.0 | 71.5% | 84.6% |
| 19 | B | 1.3 | 0.8 | 0.5 | 48.6% | 93.8% |
| 20 | C | 1.0 | 2.2 | 3.0 | 68.0% | 76.5% |

## EXAMPLE 21

This example shows the use of a continuous reactor (0.5 liter agitated glass pressure vessel with a metal top) into which all ingredients were fed continuously.

At steady state, liquid feeds of 90 ml/hour of toluene, containing 0.6 wt % of coco benzyl bis-(betahydroxypropyl) ammonium chloride; 240 ml/hour of aqueous sodium hypochlorite, containing nominally 11 wt % NaOCl plus 25 g/liter $Na_2CO_3$ and 3 g/liter NaOH, were fed concurrently with a gaseous feed of about 60 g/hour of HFP. The reactor was agitated by a paddle stirrer at 900 rpm. A three-phase liquid product was removed continuously from the reactor such that the liquid inventory in the reactor remained constant at 300 ml. Conditions were held constant at -3°C, pH 8.1, and 207 kPa (30 psig) for three hours. The three-phase liquid product was reduced to atmospheric pressure across a pressure letdown valve.

Gas and liquid phases were gravity separated and the gas phase was analyzed by gas chromatography and found to consist of 49 mole % HFP and 51 mole % HFPO. The remaining liquid was separated into organic and aqueous layers. The organic phase was analyzed and found to be 98.7% toluene.

During the third hour the weight of HFP fed was determined to be 62.45 g by weighing the feed cylinder. The gaseous reactor product was collected during the third hour by condensation in a cylinder immersed in dry ice. Condensate weighing 59.32 grams was collected with a composition of 49 mole % HFP and 51 mole % HFPO.

A material balance allowed calculation of conversion of HFP (56%) and selectivity to HFPO (82%). The recovered organic phase was analyzed and found to be 99.2% toluene. The aqueous phase contained 6600 ppm total fluoride based on combustion analysis.

## EXAMPLE 22

This continuous example is similar to Example 21, but a different phase transfer catalyst was used. At steady state, the continuous flows were 600 ml/hour of hypochlorite feed solution, which was 5.25% NaOCl solution saturated with sodium chloride and also containing 30 g/l $Na_2CO_3$; 60 ml/hour of 1% trioctyl methyl ammonium chloride catalyst in toluene; and 50 g/hour HFP.

The reactor temperature was controlled at -5°C, with a liquid volume of 375 ml. The system was operated at pH 9.6 with stirring for three hours at the flows shown above. The product gases were analyzed by gas chromatography to be 49 mole % HFPO and 51 mole % HFP. The aqueous and toluene phases were collected during the third hour and analyzed. The toluene analyzed 98.2% pure. The aqueous phase contained 6252 ppm total fluorides by the combustion technique. While the product was not collected for material balance, the conversion was calculated as 54% and the selectivity as 81%, based on product gas composition and fluorides found in the aqueous phase.

## EXAMPLE 23

In this example, a 500 ml continuous stirred glass autoclave was used. It was equipped with pumps for continuous feeding of sodium hypochlorite and solvent/catalyst solution, and a mass flow controller for continuous feeding of HFP. The volume of liquid ingredients in the reactor was maintained at 300 ml by a level sensing device controlling outflow through a letdown valve, to a collection flask. The pH of the reactor effluent was monitored by an electrode in the letdown line. The temperature was controlled at 0°C by circulating chilled glycol solution through the reactor jacket. The reactor operated at the autogenous pressure exerted by the liquid HFPO phase, about 172-241 kPa.

An aqueous solution of 4 g NaOH plus 25 g $Na_2CO_3$ dissolved in one liter of 10.2% aqueous NaOCl solution was fed to the reactor at a rate of 240 ml/hour. A solution of 0.6g of the same phase transfer catalyst used in Example 1 and 99 g of toluene was fed at 90 ml/hour. The reactor was operated for 3 hours at an average temperature of -3°C. The pH was 8.02-8.13 during the operation. Gas chromatography at 30-minute intervals showed the product gas averaged 51 mole percent (range 48-52%) HFPO and 49 mole percent HFP for the 3-hour period. Solubility data for HFP and HFPO in toluene indicate the presence of three phases under these conditions.

The weight of HFP fed during the third hour, as determined by HFP cylinder weight loss on an analytical balance, was 62.5 g. The weight of HFP/HFPO recovered by condensing gaseous product (made during the third hour) from the receiver flask in dry ice traps was 58.1 g, which analyzed 50.7 mole percent HFPO and 49.3 mole percent HFP. This material balance indicates an in-hand selectivity of 78.5% at a conversion of 54%.

Analysis for total fluorine content (4.2 g) of the aqueous phase, which contains the major by-products formed, indicated only 5.6 g HFP loss by side reactions to make aqueous by-products. If this were the only yield loss, the conversion would be 56% and the selectivity 83%.

This example shows that very good results can be obrained at -3°C and pH 8.02-8.13.

## EXAMPLE 24

In the equipment of Example 23, an aqueous solution of 45 g KOH dissolved in one liter of 10.4% aqueous NaOCl solution was fed at 200 ml/hr, a solution of 0.9 g of the same phase transfer catalyst used in Example 23 in 99 g of toluene was fed at 60 ml/hr, and HFP was fed at 60 g/hr for 3 hours. The average temperature was 1°C, and the average pH was 8.2. The composition of the product gas, as determined by gas chromatograph at 30-minute intervals, averaged 59 mole percent HFPO and 41 mole percent HFP for the entire run after the first 30 minutes.

Based on these analyses and the total fluorine found in the waste aqueous phase by analysis, the conversion was 65% and the selectivity was 78%.

At the end of 3 hours the amount of KOH in the aqueous feed was increased to 50 g/liter of 10.4% aqueous NaOCl solution and the run was continued at the above feed rates. The pH gradually increased to 9.5 at the end of 2 hours and then increased rapidly to pH 12 by the end of the third hour. The HFPO content of the gaseous product decreased gradually to 56% during the first 1.7 hours, then rapidly to 20% during the final hour. Based on product composition and total fluorine in the aqueous waste from the third hour, conversion was 28% and selectivity was 64%.

This example illustrates the higher selectivity and especially the higher conversion obtained at pH 8.2 as compared with operation at pH 10-12 under these three-liquid-phase conditions.

## EXAMPLE 25

A one-liter agitated glass autoclave was charged with the following reactants: 0.6 g of the same phase transfer catalyst used in Example 1, dissolved in 60 ml of toluene; and 600 ml of 5.25% aqueous NaOCl (equivalent to 5.0% available chlorine) containing 18 g of $Na_2CO_3$ and saturated with NaCl to prevent freezing. The reactor was cooled to -15°C and 50.0 g of HFP was added without agitation, to avoid starting the reaction while the addition of HFP was made. The reactor was sealed throughout the HFP addition and the reaction period so it remained under the autogenous pressure of the condensed fluorocarbon phase. Solubility data for HFP and HFPO in toluene indicate the presence of three phases under these conditions.

Immediately after the HFP addition the flat paddle agitator, driven magnetically, was started and run for one minute, then stopped to allow the phases to separate to stop the reaction. Then a small gas sample was removed for gas chromatographic analysis. This procedure was repeated, with agitation periods of 2, 4, 8, 16, 32, and 60 minutes, to follow the progress of the batch reaction. There was relatively little increase in the HFPO content of the gas phase during the final 60-minute period, so the reaction was discontinued, after a total agitation time of 123 minutes. The final gas composition was 75.8 mole percent HFPO and 24.2 mole percent HFP. The aqueous phase was analyzed by ion selective electrode/combustion technique, which showed that it contained 3958 ppm total fluorine, a measure of the by-products formed from HFP. Based on the analyses, the HFP conversion was 77.7% and the selectivity was 90.3%.

The temperature started at -15°C, increased to -10°C during the initial short periods of agitation, and remained in the range -10 to -15°C. The pH of the aqueous phase, measured at the end of the run, was 7.8 and the percent available chlorine remaining in the aqueous phase was 0.8%, corresponding to a conversion of NaOCl of 84%.

This example illustrates the high conversion of HFP and NaOCl and the high selectivity attainable with batch operation at relatively low pH.

**Claims**

1. A process for converting a perfluoroolefin to its epoxide comprising agitating a system of three liquid phases which are:

1) an aqueous solution comprising a sufficient amount of metal hypohalite,

2) a epoxide phase, and

3) a reaction phase comprising an organic liquid having a perfluoroolefin-epoxide partition coefficient greater than 1.3 under reaction conditions;

in the presence of a phase transfer catalyst which is soluble in the aqueous solution and the reaction phase but substantially insoluble in the epoxide phase under reaction conditions, under such reaction conditions that three phases are maintained as the reaction progresses.

2. A process according to claim 1 wherein the partition coefficient is at least 1.9.

3. A process according to claim 2 wherein the partition coefficient is at least 2.3.

4. A process according to any one of the preceding claims wherein the perfluoroolefin is hexafluoropropylene, the metal hypohalite is sodium hypochlorite and the organic liquid in the reaction phase is toluene.

5. A process according to claim 4 wherein the ratio of sodium hypochlorite to hexafluoropropylene is from 0.5:1 to 1.2:1.

6. A process according to claim 4 or 5 wherein the reaction conditions comprise reacting at a temperature from -20°C to 15°C.

7. A process according to claim 6 wherein the temperature is from -15°C to +5°C.

8. A process according to any one of claims 4 to 7 wherein the reaction conditions comprise maintaining the aqueous phase at a pH of 7 to 14.

9. A process according to claim 8 wherein the pH is from 7.5 to 9.6.

10. A process according to claim 9 wherein the aqueous phase comprises a buffer.

11. A process according to claim 10 wherein the buffer is an alkali metal carbonate.

12. A process according to any one of claims 9 to 11 wherein the pH is from 7.8 to 9.0.

13. A process according to claim 12 wherein the pH is from 8.0 to 8.5.

14. A process according to any one of the preceding claims wherein the phase transfer catalyst is a quaternary ammonium salt.

15. A process according to claim 14 wherein the catalyst is coco benzyl bis(betahydroxypropyl) ammonium chloride or trioctyl methyl ammonium chloride.

16. A process according to claim 15 wherein the phase transfer catalyst is coco benzyl bis-(betahydroxypropyl) ammonium chloride, present in an amount from 0.001 to 0.2 g/g perfluoroolefin.

17. A process according to claim 16 wherein the perfluoroolefin is hexafluoropropylene and the catalyst is present in an amount from 0.005 to 0.05 g/g hexafluoropropylene.

18. A process according to claim 17 wherein the catalyst is present in an amount from 0.008 to 0.02 g/g hexafluoropropylene.

19. A process according to any one of the preceding claims wherein the epoxide phase comprises an epoxide solvent which is substantially immiscible with the aqueous and reaction phases.

20. A process according to claim 11 wherein the epoxide solvent is a perhalogenated liquid other than the perfluoroolefin or its epoxide.